(19) Europäisches Patentamt
European Patent Office
Office européen des brevets

(11) Publication number: 0 257 887 A2

(12) **EUROPEAN PATENT APPLICATION**

(21) Application number: 87307013.0

(22) Date of filing: 07.08.87

(51) Int. Cl.⁴: **A 61 K 31/34**, A 61 K 31/35, A 61 K 31/365, A 61 K 31/12, A 61 K 31/52, A 61 K 31/47, A 61 K 31/135, A 61 K 31/165

(30) Priority: **18.08.86 US 897744**

(43) Date of publication of application: **02.03.88 Bulletin 88/9**

(84) Designated Contracting States: **AT BE CH DE ES FR GB GR IT LI LU NL SE**

(71) Applicant: **HOUSTON BIOTECHNOLOGY INCORPORATED, 3606 Research Forest Drive, The Woodlands Texas 77381 (US)**

(72) Inventor: **Lam, Dominic Man-Kit, 9 Wedgewood Glen, The Woodlands Texas 77380 (US)**
Inventor: **Varma, Rajender Singh, 333 Holly Creek Court Apartment 1514, The Woodlands Texas 77381 (US)**
Inventor: **Chiou, George C. Y., 11019 Rose Circle, College Station Texas 77380 (US)**

(74) Representative: **Harrison, David Christopher et al, MEWBURN ELLIS & CO 2/3 Cursitor Street, London EC4A 1BQ (GB)**

(54) **Ophthalmic compositions.**

(57) Alkaloids, chalcone derivatives, and fused benzo-oxaheterocycles are employed for improvement in ocular blood flow. The compounds can be administered topically or systemically to enhance the retinal and/or choroidal blood flow without having any adverse affect on the cardiovascular system.

EP 0 257 887 A2

# OPHTHALMIC COMPOSITIONS

This invention is related to compositions and techniques employed for enhancing retinal and choroidal blood flow and preventing nerve degeneration.

The retina is supplied with oxygen and nutrients by two vascular systems, one within the retina itself (central retinal artery) and one in the choroid (posterior ciliary artery). Interruption or impairment of either system leads to degeneration of the retina and ultimately to loss of vision. There are many causes that affect retinal circulation and nutrient supply. Included among diseases and conditions are retinitis pigmentosa, diabetic retinopathy, sickle cell retinopathy, hypertensive and atheroscelerotic vascular diseases, retinal vein occlusion, maculopathy, and glaucoma (LaVaio et al., (1985); U.S. Department of Health and Human Services, (1983)). By providing for enhanced blood flow or nutrient supply to the retina, particularly early in the course of the above indicated diseases, prevention or slowing of vision loss may be achieved. Any drug, given systemically or locally to the eye, that is directed toward a disease symptom but results in a decrease in ocular circulation as a side effect, will normally enhance the visual deterioration, rather than improve it.

Glaucoma serves as an illustration of the problem, where glaucoma is treated by decreasing intraocular pressure (IOP) in order to improve optic nerve, choroidal, and retinal circulation. It is found that

some patients show retinal degeneration with normal IOP (low tension glaucoma), while others have abnormally increased IOPs with no visual disturbances. This may suggest that the correlation between IOP and retinal circulation is tenuous. A drug may lower IOP and concommitantly decrease retinal and choroidal blood flow (RCBF), leading to the false conclusion that the glaucoma is under control, while in fact retinal damage continues. It is therefore of substantial interest to find drugs which increase ocular circulation as their mechanism with the resulting IOP decrease.

Relevant Literature

Xanthotoxin is reported as a photosensitizing compound which has been used in the treatment of lesions. Fitzpatrick and Pathak, J. Invest. Dermatol. (1959) 32:229. Injection of xanthotoxin coupled with UV radiation (320-400 nm) can result in generation of photoproducts within ocular tissues. Lerman et al., Invest. Ophthalmol. Vis. Sci. (1984) 25:1267. Pharmacological action of scoparone has been reported. Jamwal, et al., Ind. J. Med. Res. (1972) 60:763. Studies of natural coumarins for hypotensive activity (Joshi et al., Ind. J. Chem. (1980) 19B:495) and for coronary vasodilation (Bariana, J. Med. Chem. (1970) 13:544) have been reported. Scoparone is taught to be teratogenic. Chandhoke, Indian J. Exptl. Biol. (1979) 17:740. Biological activity of flavonoids, particularly as enzyme inhibitors, is reported by Varma et al., Science (1977) 195:205; Schwabe and Flohe, Hoppe-Seyler's Z. Physiol. Chem. (1972) 353:476; Beretz et al., Experientia (1979) 34:1054; Baumann et al., Arch Pharm., (Weinheim) (1980) 313:330; Baumann et al., Prostaglandins (1980) 20:627; Sekiya and Okuda, Biochem. Biophys. Res. Commun., (1982) 105:1090; Kostanecki et al., Ber. (1904) 37:1402 and Traditional Chinese Medicine Encyclopedia, Hsiu-Wen Fong Publ. Co., Taipei, 1985.

For a review of chalcone chemistry and biological activity, see Chemistry of Chalcones and Related Compounds, D. N. Dhar, John Wiley, N.Y., 1981. The use of 2,4,6-trihydroxychalcones as hypotensives is described in West German Offenlegungschift 2010180 (1970).

Alkaloid are known to have numerous local and systemic effects in mammals. For a discussion of the peripheral-dopamine-receptor blocking activity of bulbocapnine, see Pendleton *et al.*, *Arch. Pharmacol.* (1975) 289:171.

## SUMMARY OF THE INVENTION

Compositions and methods are provided for enhancing the retinal and/or choroidal blood flow for treatment of ocular conditions involving inadequate blood flows and nutrient supply. The compositions are characterized by containing an alkaloid having a purine or aporphine ring system or a tyramine nucleus, a chalcone (i.e., 1,3-diphenyl-2-propen-1-one) derivative, or a compound having a central fused benzo-oxaheterocycle of from 5 to 6 ring members, particularly chromanones, chromones, and benzofuran rings. The compositions may be administered topically, individually, or in combination, in a form absorbable by ocular and optic nerve tissues.

## DETAILED DESCRIPTION OF THE SPECIFIC EMBODIMENTS

Methods and compositions are provided for treating ophthalmic conditions involving inadequate retinal and/or choroidal blood flow with ophthalmic formulations comprising as an active ingredient a selected alkaloid, chalcone, or benzo-oxaheterocycle. The formulations will normally involve the active compound in a physiologically acceptable medium, which may be administered topically, intravenously or orally in a form available to retinal tissue. Compounds of particular interest are from naturally occurring medicinal plants and analogs thereof.

For the most part, the compounds of this invention that are benzo-oxaheterocycles will be chromones, chromanones, or benzofurans of at least 8 carbon atoms, having from 2 to 4 rings, which may be fused or unfused, having at least 2 oxygen atoms and not more than 6 oxygen atoms, usually not more than about 5 oxygen atoms, which will be present as oxy or oxo, usually not more than 2 being present as oxo, where the oxygen atoms will be bonded to a ring member or be a ring member.

The compounds will have at least 8, usually 9, carbon atoms (in the basic ring system) and not more than about 30 total carbon atoms, more usually not more than about 20 total carbon atoms. In addition, the glycosides of such compounds are also included, which will add from 5 to 12 carbon atoms and from 4 to 12, usually from 4 to 11, oxygen atoms to the oxygen atoms indicated above. For the most part, substituents on the ring members will be aryl, particularly oxyaryl, more particularly phenyl or oxyphenyl, usually of from 6 to 10 carbon atoms; oxy of from 0 to 3 carbon atoms, e.g., alkoxy; or divalent groups which with the vicinal ring members to which they are attached form a fused ring.

For the most part, the compounds of this first subclass will have the following formula:

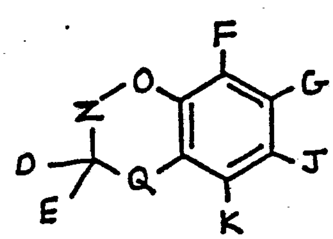

wherein:

Z is carbonyl or C(A)(B);

A is hydrogen; aryl of 6 to 10 carbon atoms (especially phenyl), having from 0 to 2 substituents, particularly oxy substituents of from 0 to 3 carbon atoms, e.g., hydroxy, methoxy, ethoxy and propoxy (-1 or -2); or alkyl of from 1 to 3, usually 1 to 2 carbon atoms, which may be substituted or unsubstituted, normally substituted, having from 0 to 1 oxy of from 0 to 3 carbon atoms, more usually hydroxy;

B is hydrogen or is taken together with D to form a double bond;

D is hydrogen or is taken together with B to form a double bond;

Q is carbonyl, a bond to form a 5-membered ring, or may be taken together with C(E) to form an ethenylene group, particularly when Z is carbonyl;

E is hydrogen; hydroxyl; aryl of from 6 to 10 carbon atoms, particuarly oxyphenyl or phenyl; or alkyl of from 1 to 3 carbon atoms, usually methyl; or may be taken together with the carbon to which it is attached and Q to define an ethenylene group;

F is hydrogen; oxy of from 0 to 3 carbon atoms, more usually of from 0 to 1 carbon atom, e.g. hydroxy or methoxy; or may be taken together with G to define a divalent radical forming a fused ring with the carbon atoms to which F and G are attached;

G is hydrogen, oxy of from 0 to 3 carbon atoms, or may be taken together with either F or J to form a 5- or 6-membered ring with the carbon atoms to which G and F or J are attached;

J is hydrogen; oxy of from 0 to 3 carbon atoms, particular hydroxy or methoxy; or an aliphatic group of from 1 to 6 carbon atoms, which may be saturated or unsaturated, particularly unsaturated, having 0 to 1 site of ethylenic unsaturation; or may be taken

together with G to form a fused ring, particularly a 5-membered ring, more particuarly a vinyloxy group to define a furan ring, where the oxygen is at G and the carbon is at J;

K is hydrogen or oxy of from 0 to 3 carbon atoms, particularly hydroxy or methoxy;

and glycosides of said compounds, including C-glycosides, especially glycosides in which the sugar moiety contains from 5 to 12 carbons, more especially those formed from mono- or disaccharides formed from 5- and/or 6-carbon sugars. Any of the hydroxyl groups may be bound to a sugar, particularly a 5- or 6-carbon sugar, more particularly a 6-carbon sugar, there being from 0 to 2 sugars per molecules, where the sugars may be bonded to different oxygen atoms of the core molecule or in tandem to form a disaccharide. The sugars of interest include glucose, rhamnose, galactose, etc.

When F and G are taken together, preferred divalent radicals provide cyclic compounds having the following formulae (in which the partial ring and brace represent the phenyl ring to which F and G are attached, along with the remainder of the molecule):

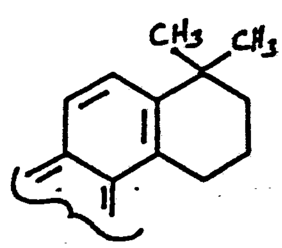

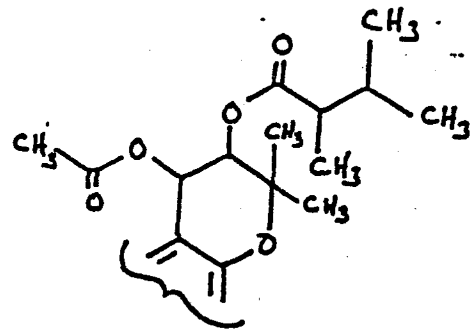

Of particular interest are the chromanones, either alpha or gamma, which will for the most part have the following formulae:

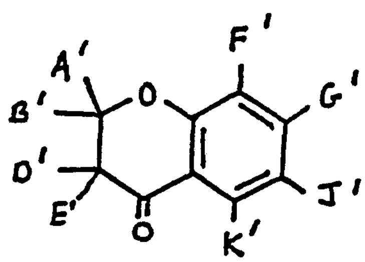

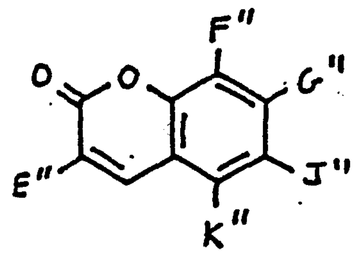

wherein:

A' is hydrogen; phenyl; oxyphenyl or dioxyphenyl, wherein the oxy groups will be of from 0 to 3 carbon atoms, more usually of from 0 to 1 carbon atom, or alkyl or hydroxyalkyl, wherein the alkyl group is of from 1 to 3 carbon atoms, particularly 1 carbon atom;

B' and D' are hydrogen or are taken together to form a double bond;

E' is hydrogen, hydroxyl, phenyl or hydroxyphenyl, particular <u>para</u>-hydroxyphenyl;

F' is hydrogen, hydroxyl, or glycosidyl, particularly glycosidyl, or may be taken together with G' to define a divalent radical as described for F and G;

G' is hydrogen, oxy of from 0 to 3 carbon atoms, particularly hydroxy or methoxy, glycosidyl, or may be taken together with J' to define an oxyvinyl group;

K' is hydrogen or oxy of from 0 to 3 carbon atoms, particular hydroxy or methoxy;

E" is hydrogen or alkyl of from 1 to 3 carbon atoms, particularly methyl;

F" is hydrogen, oxy of from 0 to 3 carbon atoms, particularly hydroxy or methoxy, or may be taken together with G" to define a divalent radical forming a fused ring where the carbons to which F" and G" are attached, where the divalent radicals are as described above for F and G;

G" is hydrogen or may be taken together with J" to form an oxyvinyl group;

J" is hydrogen, oxy of from 0 to 3 carbon atoms, particularly hydroxy or methoxy, or may be taken together with G";

K" is hydrogen or oxy of from 0 to 3 carbon atoms, particularly hydroxy.

The subject compounds may also exist in alternative forms such as a noncyclic analog, e.g., where an ω-hydroxypentene-1 may exist as the linear form or the cyclic ether or a ketone may exist as the carbonyl or the enol.

Specific compositions of interest include astragalin, kaempferol, coryfolin, flavone, khellinin, puerarin, scoparone, tanshinone, visnadin, and xanthotoxin.

For the most part, the compounds of this invention that are related to chalcones will be chalcone derivatives or hydrogenated chalcone derivatives (i.e., derivatives of 1,3-diphenyl-2-propen-1-one or

the propanone analogue) having hydrogens or $C_1$ or $C_4$ alkyl groups as substituents on the carbon chain linking the two phenyl groups and typical substituents found on aromatic rings being present on the two phenyl groups themselves. Preferred are hydrocarbyl groups, substituted hydrocarbyl groups, hydroxy groups, hydrocarbyloxy groups, and substituted hydrocarbyloxy groups. Substituents on hydrocarbyl and hydrocarbyloxy groups are typically oxygen-containing functional groups such as hydroxy and oxo groups, preferrably no more than two substituents per hydrocarbyl group with no more than one substituent being present per carbon unless present in the form a carboxyl group. Esters and ethers are permitted.

For the most part, the compounds of this subclass will have the following formula:

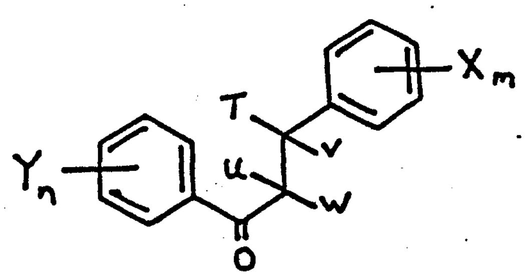

wherein:

T, U, V, and W independently represent H or a $C_1$-$C_4$ alkyl group or T and U together represent a C-C bond;

each X and Y independently represents R, OH, or OR, wherein each R independently represents a $C_1$-$C_6$ alkyl group or a $C_2$-$C_6$ alkenyl or alkynyl groups substituted with 0-2 oxygen-containing functional groups (especially oxy or oxo substituents) or two adjacent X or two adjacent Y together with the atoms of the phenyl ring form a cyclic hydrocarbyl group (optionally containing oxygen) with 5 or 6 ring atoms; and

m and n independently represent an integer from 0-4.

Preferred are chalcones, as opposed to hydrogenated chalcones, in which T and U together represent a C-C bond. V and W are preferrably methyl or H, most preferrably H. Also preferred are compounds having two or fewer hydroxy groups in the ring adjacent to the carbonyl.

The number of substituents on the two phenyl rings is selected in order to provide a stable compound. Accordingly, m and n therefore are generally 1, 2, or 3 with m + n normally falling in the range from 2-6. Hydroxy groups present at the 2, 3 or 4 position of the two phenyl rings are preferred, especially the 4 position alone or in pairs at the 2 and 4 or 3 and 4 positions. In describing substituents present in the rings, the notations $Y_1$, $Y_2$, etc. are used to represent the first substituent in the ring, the second substituent, and so on. Thus, two parahydroxy substituents would be referred to as $Y_1$ = 4-OH and $X_1$ = 4-OH, in which the 4 refers to the position in the ring. The preferred position for a hydrocarbyl substituent in each ring is the 3- position of the ring. A preferred grouping is $Y_1$ = 4-OH, $Y_2$ = 2-OH, $Y_3$ = H or R, $X_1$ = 4-OH.

Preferred hydrocarbyl substituents are the isopentyl and isopentenyl groups and the oxy-substituted derivatives thereof. The double bond in the isopentenyl group joins carbons 2 and 3 of this substituent. Oxy-substituents, particularly hydroxy substituents, can be at any replaceable hydrogen. Cyclic substituents form by the interaction of an

oxygen-containing isopentyl or isopentenyl group with an adjacent oxygen to form an ether linkage represent a preferred class of cyclic substituents. Examples of two such cyclic systems are set forth in the following formulae (in which the partial phenyl rings represent one of the two phenyl rings of a chalcone):

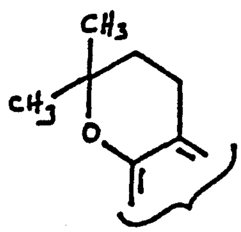

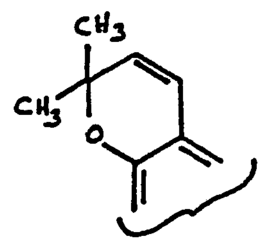

Substituents that can interact to form 5-membered oxygen-containing rings (especially furan and dihydrofuran rings) are also preferred. Other cyclic substituents include alkylenedioxy substituents, espcially methylenedioxy substituents, at adjacent phenyl ring positions.

Of particular interest are the chalcones having the following formulae:

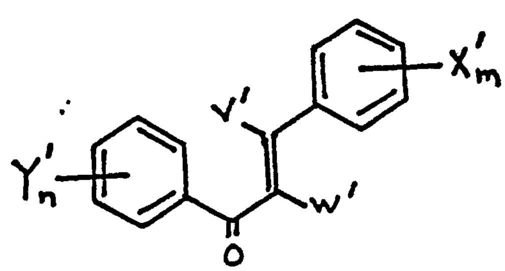

wherein:

V' and W' independently represent H or methyl, preferrably H;

each X' and Y' independently represents R', OH, or OR', wherein each R' independently represents a $C_1$-$C_6$ alkyl group or a $C_2$-$C_6$ alkenyl or alkynyl group or two adjacent X' or Y' together with the atoms of the phenyl ring to which they are attached form a cyclic oxahydrocarbyl group with 5 or 6 ring atoms with the oxygen being adjacent to the phenyl ring; and

m and n independently represent an integer from 1 to 3.

Particularly preferred are compounds in which m + n = 3 or 4, $Y_1$ is 4-OH, $X_1$ is 4-OH, $Y_2$ is R or H, and $Y_3$ is 2-OH

Specific compounds of interest include corylifolinin and isoliquiritigenin.

In addition, various derivatives of compounds specified above can readily be prepared and are considered equivalent thereto. These specifically include esters formed by the reaction of a carboxcylic acid group with a hydroxyl of the pricipal compound, particularly ethers formed from acetic acid. Other equivalent derivatives include glycosides formed by the reaction of a naturally occurring 5- or 6-carbon sugar (or a disaccharide formed from such sugars) with a hydroxyl group on the principal compound.

Compounds of the invention can readily be produced by standard synthetic techniques. Chalcone itself can be prepared by the action of sodium hydroxide on an alcoholic solution of benzaldehyde and acetophenone (an aldol condensation). By selecting substituted benzaldehyde and acetophenone derivatives to provide the desired substituents in the product molecule, the various compounds of the invention can readily be synthesized. Synthesis of such compounds is described in Jain et al., Indian J. Chem. (1969) 7:1072. Isolation and properties of natural compounds having the chalcone structure are described in The Flavanoids: Advances in Research, Harborne and Mabry, eds. Chapman and Hall, Ltd., London (1982), pp. 313-410.

For the most part, the compounds of this invention that are alkaloids will be substituted purines, aporphines, and tyramines. The purine and aporphine ring systems are shown below.

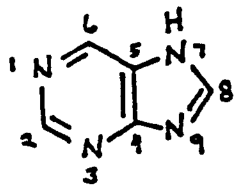

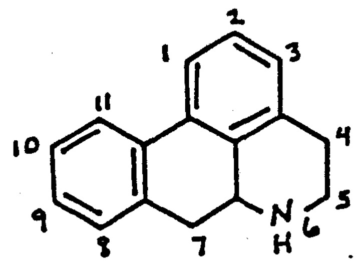

When the alkaloid is a purine, an amino or substituted amino group will typically be present at the 6 position and an alkyl, alkenyl, or alkynyl substituent present at the 3 position. Substituents for the amino group include $C_1$-$C_6$ alkyl, $C_2$-$C_6$ alkenyl or alkynyl, $C_1$-$C_6$ alkanoyl, and combinations thereof, usually not more than one alkanoyl substituent being present on a single nitrogen. The substituent on N3 typically has 6 or fewer carbon atoms, 0 or 1 carbon-carbon double bond, and 0-2 hydroxy groups. Other substituents, when present, are generally similar to the substituents that may be found on N3 and are generally located, if present, on the nigrogens at positions 7 and 9. A typical compound is triacanthine, which has an amino substituent at position 6 and an isopent-2-en-1-yl substituent on the nitrogen at the 3 position. Isopent-2-en-1-yl substituents (usually no more than one per molecule) can also be present at positions 7 or 9 or on an amino substituent at position 6. Hydroxy-substituted isopentenyl and isopentyl substituents are also preferred.

When the ring structure of the alkaloid is an aporphine ring, substituents are typically present at the 1, 2, 6, 10 and/or 11 positions, with substituents at other positions being relatively less frequent. Typical substituents are hydroxy, R, and OR, in which R represents a $C_1$-$C_6$ alkyl group or a $C_2$-$C_6$ alkenyl or alkynyl group substituted with 0-2, preferrably 0, oxygen-containing functional groups, such as oxo and hydroxy groups. Small alkyl substituents, such as methyl, ethyl, and the various propyl and butyl substituents are preferred as R groups. Hydroxy or OR substituents are the most common groups at positions 1, 2, 10 and 11 of the aporphine ring. Alkylenedioxy bridging groups, such as the methylenedioxy group, can link adjacent positions, namely the 1 and 2 positions, 10 and 11 positions, and 11 and 1 positions. Hydroxy groups may be acylated with a carboxylic acid, typically a $C_1$-$C_6$ alkanoyl or benzoic acid compound, preferrably acetyl. Examples of compounds of the invention include bulbocapnine, magnoflorine, apocodeine, apomorphine, and aporeine.

When the alkaloid is a tyramine, the compound will have the basic tyramine structure, namely 4-(2-aminoethyl)phenol. Any hydrogen can be substituted by a $C_1$-$C_4$ alkyl group, particularly methyl. Preferred are substituents on the oxygen and nitrogen atoms. Derivatives formed by the reaction of a carboxylic acid with the amino or hydroxy group of tyramine are also useful, particularly acetyl derivatives. Methyl tyramine is a preferred compound. Salts, particularly acid addition salts such as HCl salts, of the amino group are considered to be equivalent to the parent compounds.

A number of preferred compounds of the invention are alkaloids of a first formula

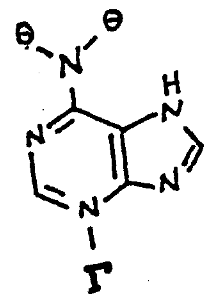

wherein each Θ independently represents a $C_1$-$C_6$ hydrocarbyl group or a $C_1$-$C_6$ carboxylic acid residue, and Γ represents a $C_1$-$C_6$ alkyl group or a $C_2$-$C_6$ alkenyl or alkynyl group substituted with 0-2 oxygen-containing functional groups; a second formula:

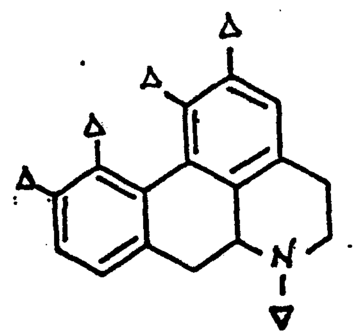

wherein ∇ represents a $C_1$-$C_6$ alkyl group or a $C_2$-$C_6$ alkenyl or alkynyl group and each Δ independently represents H, OH, R, or OR, wherein each R independently represents a $C_1$-$C_6$ alkyl group, the $C_2$-$C_6$ alkenyl or alkynyl group, or a $C_1$-$C_6$ carboxylic acid residue, or two Δ at adjacent positions together can represent a hydrocarbyldioxy group which together with the remaining atoms of the phenyl ring to which said substituents are attached form a heterocyclic ring having from 5 to 7 atoms in said ring; or a third formula:

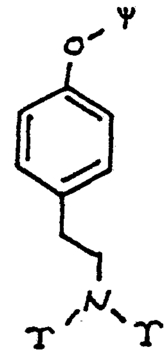

in which Ψ and T independently represent a $C_1$-$C_4$ alkyl group or the acyl residue of a carboxylic acid containing from 1 to 6 carbon atoms, with the proviso that no more than 1 T is a carboxylic acid residue (each T operating independently). Salts of these compounds are considered to be equivalent to the parent compound.

Compounds in the invention can readily be produced by standard synthetic techniques or purchased commercially. Because of the importance of both the purine ring system (present and nucleic acids and numerous other biochemical substances) and the aporphine ring system (which is readily derived from morphine and its many analogs), the total synthesis and/or partial synthesis from natural products of these compounds have been extensively studied. See, for example, any addition of the Merck Index, which lists numerous techniques for all of the compounds individually specified herein.

Some of the subject compounds besides providing for enhanced retinal and choroidal blood flow may have side effects which will limit the nature of the hosts which may enjoy the benefits of the subject compounds. Thus, compounds which may be suspected of being mutagenic may have certain restricted applications, particularly in relation to pregnant women, individuals susceptible to mutagenesis, and the like.

Some of the compounds, for example, apomorphine and related aporphines, are known to be emetics.

The subject compounds are formulated to provide for treatment and/or prevention of retinal and optic nerve degeneration, including glaucoma, resulting from insufficient blood supply to retina, choroid and optic nerve tissue. In some instances, the compound may also serve to increase coronary blood flow to benefit angina pectoris, an important consideration with middle-aged or older patients. The compounds are formulated in conventional formulations in a form absorbable by ocular and optic nerve tissue.

The formulations can be conveniently comprised of one or more active agents and mixed with a physiologically acceptable carrier, particular liquid or ointment, where the carrier may be an organic or inorganic carrier. These carriers will vary depending upon the manner of application. Physiologically acceptable carriers include water, phosphate-buffered saline, saline, aqueous solvents, where water is mixed with lower alkanols, vegetable oils, polyalkylene glycols, petroleum-based jelly, ethyl cellulose, ethyl oleate, carboxymethyl cellulose, polyvinylpyrrolidone, isopropyl myristate and other conventional acceptable carriers and additives.

Additives may include substances which serve for emulsifying, preserving, wetting agents, bodying agents, and the like which are employed conventionally in pharmaceutical preparations. Examples of such substances are polyethylene glycols 200-600, carbowaxes 1,000-10,000, antibacterial components such as quaternary ammonium compounds, phenylmercuric salts, e.g. thimerosal, methyl and propyl paraben, benzyl alcohol, phenethanol, etc. Other ingredients may include salts or buffering agents such as sodium chloride, sodium borate, sodium phosphate, sodium acetate, gluconate, and the like, where the pH will be maintained approximately neutral to mildly acidic, generally in the range of about 4.5 to 7.5.

Other additives which may find use include various compounds having wetting properties, chelating agents, or surfactant properties, either ionic or nonionic, such as sorbitan monolaurate, triethanolamine oleate, polyoxyethylenesorbitan monopalmitate, dioctyl sodium sulfosuccinate, monothioglycerol, thiosorbitol, ethylenediaminetetraacetic acid, etc.

Desirably, the formulation will be isotonic and various salts or other additives may be employed to achieve an isotonic composition.

Besides liquids and ointments, solids may be employed which may be used as solid inserts. The solid insert will be in a form suitable for insertion into the cul-de-sac of the eye. The active ingredient may be included with a non-bioerodible insert, i.e., an insert which remains essentially intact during dispensing of the drug; or a bioerodible insert, i.e., one that dissolves or disintegrates, particularly by the effect of lacrimal fluids. Suitable inserts are described in U.S. Patent No. 4,521,414, which disclosure is incorporated in by reference.

The dosage of the active ingredient will vary widely, depending upon the nature of the active ingredient and the manner in which the active ingredient is dispensed. Usually, for systemic administration, the active ingredient will be initially administered in the general range of 0.5-50 mg/kg of host weight, more usually from about 0.5-20 mg/kg of host weight. The dose can be adjusted according to the effect that the initial dose has on the host. For topical administration, the concentration of eye solutions will generally range from about 0.01-5%, more usually from about 0.1-0.5% by weight or an equivalent weight ratio with a solid medium.

The following examples are offered by way of illustration and not by way of limitation.

## EXPERIMENTAL

### EXAMPLE 1

### Measurement of Retinal and Choroidal Blood Flow (RCBF) with Laser Doppler Method

The laser Doppler method is based on the Doppler principle to monitor the perfusion of red blood cells in the microcirculatory bed of the retina and choroid (Goldman, Current Laser Technology in Medicine and Surgery (Springer, 1981) Ch. 5). Basically, the frequency of light back-scattered from moving red blood cells is shifted (Doppler shift) in proportion to the velocity of cells. A beam of concentrated light is focused onto the retina and choroid. The reflected light is guided through an optical fiber onto a photodetector. The flow calculation is dependent on the average velocity and the density of the cells in the measured volume. The resulting photocurrent, which contains the desired information on the speed of red blood cells is processed by means of a frequency-to-voltage converter. The output of the processing unit can be displayed both digitally and as a record tracing.

New Zealand white female rabbits weighing 3.0-3.5 kg were anesthetized with 30 mg/kg i.v. pentobarbital sodium followed 1 hr later with 0.5 g/kg i.v. urethane. Additional urethane was given throughout the experiment depending on the depth of anesthesia. With these anesthetics the eyes were immobilized sufficiently for measurement of retinal and choroidal blood flow using a laser Doppler (LD 5000 Capillary perfusion Monitor, Med Pacific Corporation, Seattle, WA). The femoral artery and vein were cannulated, respectively, for monitoring systemic blood pressure (RP-1500

Pressure Transducer and Model IVA Physiograph, Narco Biosystems, Houston, TX) and for injection of urethane and drugs. Heart rates were counted directly from the blood pressure pulse recordings. Drug solutions were adjusted to 1.5 ml for injection and were injected either slowly over a period of 60 sec or quickly as a pulse. Control experiments were done by injecting the same volume of saline. The experiment was run for at least 60 min after drug administration and readings of RCBF, systemic blood pressure and heart rate were taken at 10 min intervals.

Area under curve (AUC) was computed with a Hipad Digitizer (Houston Instrument) connected to an IBM Personal Computer XT.

Using this procedure, a comparative compound of unrelated structure, tetramethylpyrazine (TMP; 10 mg/kg i.v.), increased retinal blood flow markedly (52%) whereas L-timolol decreased it significantly (-17%). Tanshinone (SMI) had a tendency to increase RBF (14%) but was not statistically significant. These data validate the ability of this technique to detect changes in blood flow (RCBF).

## EXAMPLE 2

### Measurement of Retinal and Choroidal Blood Flow-Modified Procedure: Benzo-oxaheterocycles

The retinal and choroidal blood flow was measured with the laser Doppler method for additional compounds using a somewhat modified procedure as described below. The procedure was modified from that described by Riva and Feke, In: Current Laser Technology in Medicine and Surgery (ed. L. Goldman), Springer-Verlag, New York, 1981, Chapter 13.

New Zealand white female rabbits weighing 2.0 - 2.5 kg were anesthetized with 35 mg/kg i.v. pentobarbital sodium. A tracheotomy was performed and the animal was connected to a respirator (NARCO V. 5 kg).

A dose of decamethonium bromide (20 mg/ml in 0.9% saline), 2-3 mg, was administered via a marginal ear vein. The femoral artery and vein were cannulated, respectively, for monitoring systemic blood pressure (24005 Recorder, GOULD electronics, Cleveland, Ohio) and for infusion of anesthetic and muscle relaxant during the course of the experiment. Heart rates were counted directly from the blood pressure pulse recordings. A constant infusion of anesthetic and muscle relaxant (15 mg/ml pentobarbital sodium, 10 mg/ml decamethonium bromide on 0.9% saline) was performed at a rate of 10-12 mg/kg/hr pentobarbital sodium and 7-10 mg/kg/hr decamethonium bromide in order to maintain blood pressure stability and cessation of eye movement for the course of the experiment. The animal was placed in a stereotactic headholder and the fiber optic probe from a laser (LD 500 Capillary Perfusion Monitor, MEDPACIFIC Corporation, Seattle, WA) was positioned near the eye such that nominal flow measurements could be attained. Once the animal had stablized (no radical changes in blood pressure or flow for a half hour or longer), a baseline reading was taken of blood pressure, heart rate, DC (light level), and flow signal. Immediately following, the drug under investigation was administered (topically 0.25% in appropriate carrier) and the same parameters were read every 20 minutes for as long as the animal remained stable. Control experiments were done by using a topical application of the appropriate carrier and proceeding as above.

A graph was plotted of flow (Y-axis) related to time (X-axis). The baseline flow was represented as a discrete value plotted as a straight line across the entire time span of the graph and the experimental flow values were plotted as points every twenty minutes during the course of the experiment. Individual points may be above, below or on the baseline itself. Each point was then connected by lines to form a jagged curve.

The amount of space under the baseline (measured from the baseline value to zero on the Y-axis times the value from zero on the X-axis to the end of the time measurement) was compared to the space under the curve (Area Under Curve, AUC). If the AUC is greater than the area under the baseline, this would signify an increasing effect on flow by the drug being tested. AUC less than the area under the baseline represents a negative effect. The values presented represent a percentage increase over the baseline value in each experiment reported.

The AUC was computed with a Hipad Digitizer (Houston Instrument) connected to an IBM Personal Computer XT.

The following are the results obtained as to the systemic cardiovascular blood pressure, heart rate and the retinal and choroidal blood flow for a number of the subject compounds:

Effects of 0.25% Scoparone on RCBF

| |
|---|
| 57.58% |
| 37.97% |
| 22.73% |
| 97.39% |
| 35.53% |
| 50.25 ± 13.04% |

Effects of 0.25% Flavone on RCBF

| |
|---|
| 31.6% |
| 96.7% |
| 6.3% |
| 106.5% |
| 94.6% |
| 92.5% |
| 71.4 ± 17.0% |

Effects of 0.25% Kaempferol on RCBF

| |
|---|
| 57.21% |
| 43.40% |
| 29.07% |
| 32.88% |
| 67.06% |
| 45.92 ± 7.19% avg. |

Effects of Xanthotoxin on RCBF

| 0.05% | 0.1% |
|---|---|
| 45.4% | 91.6% |
| 127.2% | 62.4% |
| 48.2% | |
| 57.2% | 59.6% |
| 69.5 ± 19.4 avg. | 72.2 ± 10.2% avg. |

Effects of 0.25% Kaempferol on Cardiovascular Systems

| Blood Pressure (mmHg) | | | Heart-Rates (Beats/min) | | |
|---|---|---|---|---|---|
| Control | Treated | | Control | Treated | |
| 0 Min | 60 min | 120 min | 0 Min | 60 min | 120 min |
| 73.8 ± 6.1[a] / 47.4 ± 4.9 | 77.6 ± 8.8 / 50.0 ± 7.8 | 82.4 ± 9.6 / 52.6 ± 8.5 | 242±19 | 247±22 | 247±22 |

[a]Systolic P/Diastolic P, Mean ± SEM, N = 5

Effects of 0.25% Scoparone on Cardiovascular Systems

| Blood Pressure (mmHg) | | | Heart-Rates (Beats/min) | | |
|---|---|---|---|---|---|
| Control | Treated | | Control | Treated | |
| 0 Min | 60 min | 120 min | 0 Min | 60 min | 120 min |
| 57.6 ± 4.7[a] / 36.6 ± 3.5 | 62.4 ± 6.3 / 40.0 ± 4.3 | 55.6 ± 3.7 / 35.6 ± 2.6 | 218±15 | 226±16 | 216±15 |

[a]Systolic P/Diastolic P, Mean ± SEM, N = 5

Effects of 0.25% Flavone on
Cardiovascular Systems

| Blood Pressure (mmHg) | | | Heart-Rates (Beats/min) | | |
|---|---|---|---|---|---|
| Control | Treated | | Control | Treated | |
| 0 Min | 60 min | 120 min | 0 Min | 60 min | 120 min |
| 66.3 ±11.1[a]<br>43.0 ± 7.7 | 70.7 ±11.6<br>46.0 ± 8.0 | 50.5 ± 7.6<br>32.7 ± 5.2 | 224±12 | 226±14 | 220± 9 |

[a]Systolic P/Diastolic P, Mean ± SEM, N = 6

Effects of 0.1% Xanthotoxin on
Cardiovascular Systems

| Blood Pressure (mmHg) | | | Heart-Rates (Beats/min) | | |
|---|---|---|---|---|---|
| Control | Treated | | Control | Treated | |
| 0 Min | 60 min | 120 min | 0 Min | 60 min | 120 min |
| 74.7 ±14.7[a]<br>51.3 ±11.4 | 80.3 ±15.0<br>37.7 ± 4.5 | 72.7 ± 9.9<br>46.0 ± 7.2 | 236±33 | 236±28 | 236±26 |

[a]Systolic P/Diastolic P, Mean ± SEM, N = 3

Effects of 0.05% Xanthotoxin on
Cardiovascular Systems

| Blood Pressure (mmHg) | | | Heart-Rates (Beats/min) | | |
|---|---|---|---|---|---|
| Control | Treated | | Control | Treated | |
| 0 Min | 60 min | 120 min | 0 Min | 60 min | 120 min |
| 67.8 ± 3.8[a]<br>47.3 ± 2.6 | 72.8 ± 4.4<br>48.8 ± 2.5 | 73.5 ± 6.7<br>49.5 ± 6.2 | 252±14 | 258± 6 | 270±14 |

[a]Systolic P/Diastolic P, Mean ± SEM, N = 4

## EXAMPLE 3

### Measurement of Retinal and Choroidal Blood Flow-Modified Procedure: Chalcone Analogues

The retinal and choroidal blood flow was measured with the laser Doppler method as described above for Example 2.

The following are the results obtained as to the systemic cardiovascular blood pressure, heart rate and the retinal and choroidal blood flow for a number of the subject compounds:

Isoliquiritigenin (I)

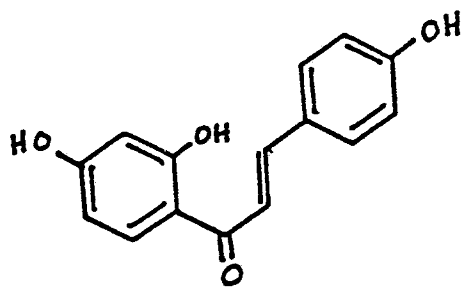

**Effect of 0.25% I on RCBF**

128.4%
50.6%
132.5%
103.8 ± 26.6%

**Effects of 0.25% I on Cardiovascular Systems**

| Blood Pressure (mm Hg) | | | Heart Rates (Beats/Min) | | |
|---|---|---|---|---|---|
| Control | Treated | | Control | Treated | |
| 0 Min | 60 Min | 120 Min | 0 Min | 60 Min | 120 Min |
| 99.3±0.7[a] / 71.3±2 | 92.3±6.7 / 67.0±3.6 | 99.3±9.7 / 70.7±7.4 | 276±2 | 264±7 | 276±12 |

[a]Systolic P/Diastolic P, Mean ± SEM, N = 3

EXAMPLE 4

Measurement of Retinal and Choroidal Blood Flow-Modified Procedure: Alkaloids

The retinal and choroidal blood flow was measured with the laser Doppler method as described in Example 2.

The following are the results obtained as to the systemic cardiovascular blood pressure, heart rate and the retinal and choroidal blood flow for a number of the subject compounds:

Triacanthine

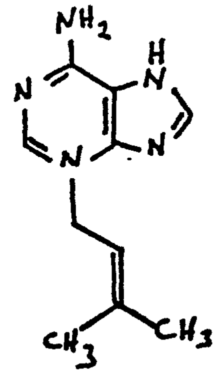

Effects of 0.25% Triacanthine on RCBF

| |
|---|
| 18.0% |
| 73.1% |
| 35.8% |
| -24.1% |
| 79.6% |
| 53.4% |
| 39.3 ± 13.5% |

Bulbocapnine

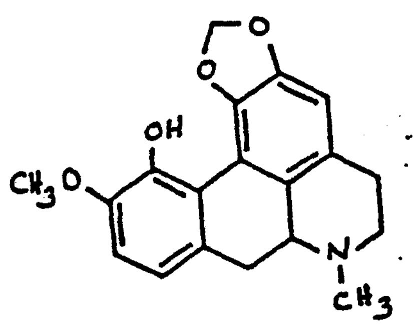

Effects of 0.25% Bulbocapnine on RCBF

| |
|---|
| 34.5% |
| 101.5% |
| 157.7% |
| 45.1% |
| 84.7 ± 28.4% |

Effects of 0.25% Bulbocapnine on Cardiovascular Systems

| Blood Pressure (mm Hg) | | | Heart Rates (Beats/Min) | | |
|---|---|---|---|---|---|
| Control | Treated | | Control | Treated | |
| 0 Min | 60 Min | 120 Min | 0 Min | 60 Min | 120 Min |
| 83.8±9.7[a] / 55.8±7.2 | 85.3±8.9 / 57.0±6.0 | 89.0±5.8 / 55.5±5.3 | 252±16 | 246±12 | 234±15 |

[a]Systolic P/Diastolic P, Mean ± SEM, N = 4

N-Methyltyramine

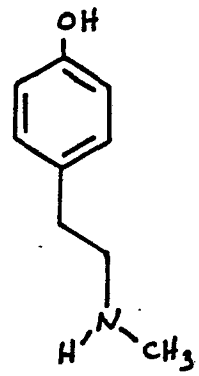

Effects of 0.25% Methyltyramine on RCBF

32.5%
121.4%

---

77.0 ± 44.5%

Effects of 0.25% N-Methyltyramine on RCBF

| Blood Pressure (mmHg) | | | Heart-Rates (Beats/min) | | |
|---|---|---|---|---|---|
| Control | Treated | | Control | Treated | |
| 0 Min | 60 min | 120 min | 0 Min | 60 min | 120 min |
| 103 ± 9[a] / 63 ± 12 | 108 ± 20 / 67 ± 22 | 105 ± 10 / 64 ± 18 | 264±24 | 264±24 | 252±12 |

[a]Systolic P/Diastolic P, Mean ± SEM, N = 5

It is evident from the above results that the compounds of the subject invention provide for substantially enhanced retinal and choroidal blood flow, while at the same time having no adverse effect on the systemic blood pressure and heart rate, with only moderate fluctuations, if any fluctuation at all. Thus, the compounds provide for unique results in not affecting the general cardiovascular system, while substantially enhancing retinal and choroidal blood flow. The subject compounds may therefore be used in the treatment of a wide variety of ophthalmological diseases associated with reduced retinal and choroidal blood flow and/or nutrient supply. The subject compounds can be administered to the host in a wide variety of ways, topically or systemically, so as to provide for the desired improvement in retinal and choroidal blood flow.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto .

WHAT IS CLAIMED IS:

1. A topical ophthalmic composition comprising (1) at least one compound of a first formula:

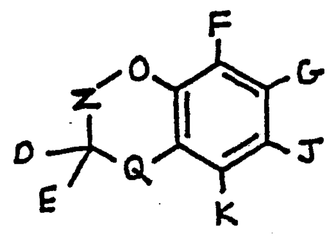

wherein:

Z is carbonyl or C(A)(B);

A is hydrogen, aryl of from 6 to 10 carbon atoms having from 0 to 2 oxy substituents of from 0 to 3 carbon atoms, or alkyl of from 1 to 3 carbon atoms having from 0 to 1 oxy substituent of from 0 to 3 carbon atoms;

B is hydrogen or is taken together with D to form a double bond;

D is hydrogen or is taken together with B to form a double bond;

Q is carbonyl, a bond to form a 5-membered ring, or may be taken together with C(E) to form an ethenylene group;

E is hydrogen, hydroxyl, aryl of from 6 to 10 carbon atoms, alkyl of from 1 to 3 carbon atoms, or may be taken together with the carbon atom to which it is attached and Q to define an ethenylene group;

0257887

F is hydrogen, hydroxyl, aryl of from 6 to 10 carbon atoms, alkyl of from 1 to 3 carbon atoms, or may be taken together with G to define a divalent radical forming a fused ring with the carbon atoms to which F and G are attached;

G is hydrogen, oxy of from 0 to 3 carbon atoms, or may be taken together with either F or J to form a 5- or 6-membered ring with the carbon atoms to which G and F or J are attached;

J is hydrogen, oxy of from 0 to 3 carbon atoms, an aliphatic group of from 1 to 6 carbon atoms, or may be taken together with G to form a fused ring; and

K is hydrogen or oxy of from 0 to 3 carbon atoms;

said compound having from 0 to 2 glycosidyl groups linked to hydroxy groups;

a second formula

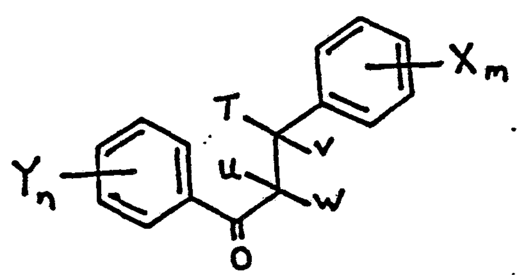

wherein:

T, U, V, and W independently represent H or a $C_1$-$C_2$ alkyl group or T and U together represent a C-C bond;

each X and Y independently represents R, OH, or OR, wherein each R independently represents a $C_1$-$C_6$ alkyl group or a $C_2$-$C_6$ alkenyl or alkynyl group substituted with 0-2 oxygen-containing functional groups or two adjacent X or two adjacent Y together with the atoms of the phenyl ring from a cyclic hydrocarbyl group optionally containing oxygen with 5 or 6 ring atoms; and

m and n independently represent an integer from 0 to 4;

a third formula

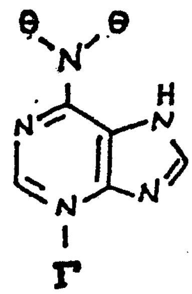

wherein each Θ independently represents a $C_1$-$C_6$ hydrocarbyl group or a $C_1$-$C_6$ acyl residue of a carboxylic acid, and Γ represents a $C_1$-$C_6$ alkyl group or a $C_2$-$C_6$ alkenyl or alkynyl group substituted with 0-2 oxygen-containing functional groups;

a fourth formula

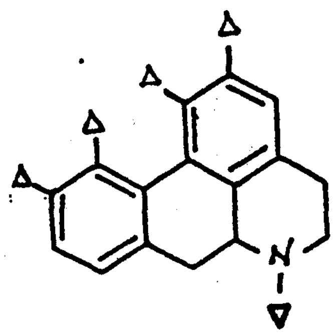

wherein ∇ represents a $C_1$-$C_6$ alkyl group or a $C_2$-$C_6$ alkenyl or alkynyl group and each Δ independently represents H, OH, R, or OR, wherein each R independently represents a $C_1$-$C_6$ alkyl group, a $C_2$-$C_6$ alkenyl

or alkynyl, or a $C_1$-$C_6$ acyl residue of a carboxylic acid, or two W at adjacent positions together can represent a hydrocarbyldioxy group which together with the remaining atoms of said fourth formula form a heterocyclic ring having from 5 to 7 atoms in said ring; or a fifth formula:

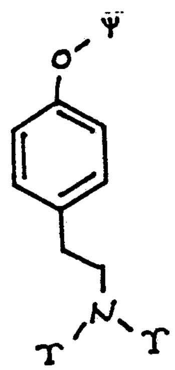

wherein Ψ and T independently represent a $C_1$-$C_4$ alkyl group or the acyl residue of a carboxylic acid containing from 1 to 6 carbon atoms.

2. A composition according to Claim 1, wherein said compound is of the formula:

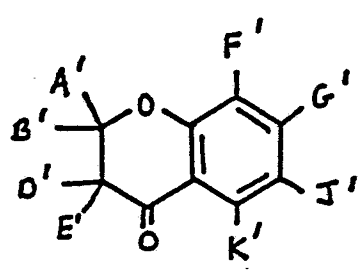

wherein:

A' is hydrogen, phenyl, oxyphenyl or dioxyphenyl, alkyl or hydroxyalkyl of from 1 to 3 carbon atoms, and the oxy group is of from 0 to 1 carbon atom;

B' and D' are hydrogen or are taken together to form a double bond;

E' is hydrogen, hydroxyl, phenyl or hydroxyphenyl;

F' is hydrogen or hydroxyl;

G' is hydrogen, oxy of from 0 to 3 carbon atoms, or may be taken together with J' to define an oxyvinyl group; and

K' is hydrogen or oxy of from 0 to 3 carbon atoms.

3. A composition according to Claim 1, wherein said compound is astragalin, kaempferol, coryfolin, flavone, khellinin, puerarin, scoparone, tanshinone, visnadin or xanthotoxin.

4. A composition according to Claim 3, wherein said compound is flavone.

5. A composition according to Claim 1, wherein said compound is of the formula:

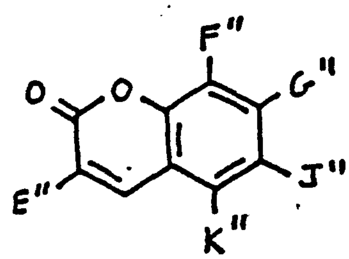

wherein :

E" is hydrogen or alkyl of from 1 to 3 carbon atoms;

F" is hydrogen, oxy of from 0 to 3 carbon atoms, or may be taken together with G" to define a divalent radical of tanshinone or visnadin, wherein E" is hydrogen;

G" is hydrogen or is taken together with F" to define a divalent radical or with J" to form an oxyvinyl group;

J" is hydrogen, oxy of from 0 to 3 carbon atoms or may be taken together with G";

K" is hydrogen or oxy of from 0 to 3 carbon atoms.

6. A composition according to Claim 1, wherein said compound is of the formula:

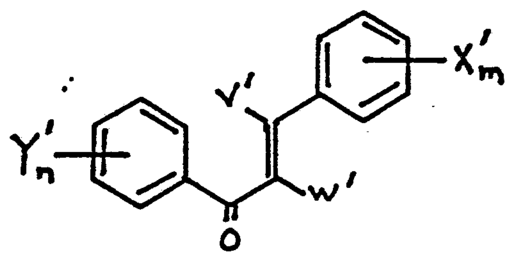

wherein:

V' and W' independently represent H or methyl;

each X' and Y' independently represents R', OH, or OR', wherein each R' independently represents a $C_1$-$C_6$ alkyl group or a $C_2$-$C_6$ alkenyl or alkynal group; and

m and n independently represent an integer from 0 to 3.

7. A ~~method~~ composition according to Claim 6, wherein said compound is isoliquiritigenin.

8. The composition of Claim 1, wherein said alkaloid has said third formula.

9. The composition of Claim 8, wherein said alkaloid is triacanthine.

10. The composition of Claim 1, wherein said alkaloid has said second formula.

11. The composition of Claim 10, wherein V is hydrogen or methyl and each W is independently selected from the group consisting of H, OH, $OCH_3$, and $OCOCH_3$, or to adjacent W together represent $OCH_2O$.

12. The composition of Claim 1, wherein said compound is bulbocapnine, apocodeine, apomorphine, or aporeine.

13. A composition according to Claim 1, wherein said composition comprises from about 0.01% to 10% of said compound.

14. A composition according to Claim 1, wherein said ophthalmic carrier is an isotonic aqueous solution having a pH of from about 4.5 to 7.5.

15. A composition according to Claim 1 in unit dosage form, where each unit comprises from about 0.005 to 5mg of said compound.

16. A solid ocular insert comprising (1) a shaped ophthalmologically acceptable polymer containing at least one compound of Claim 1 in a form absorbable by ophthalmic and optic nerve tissue, and (2) a fluid

0257887

ophthalmic carrier; said alkaloid being present in said composition in an amount effective to increase blood flow to ocular neural tissues.

17. The use of any of the compounds given and defined in claim 1 in ophthalmology.